# EUROPÄISCHE PATENTSCHRIFT

(11) **EP 0 915 079 B1**
(45) Veröffentlichungstag und Bekanntmachung des Hinweises auf die Patenterteilung: **06.08.2003**
(21) Anmeldenummer: 98120071.0
(22) Anmeldetag: 23.10.1998
(51) Int. Cl.: C07C 69/007, C07C 69/025, C07C 69/24, C07C 69/533, C07C 67/475

(54) **8-Ocimenylester und ihre Verwendung als Riech- und Aromastoffe**
8-Occimenylesters and their use as aroma and perfume
8-Occiménylesters et leur utilisation comme arome ou comme parfum

(30) Priorität: 05.11.1997 DE 19748774
(43) Veröffentlichungstag der Anmeldung: 12.05.1999
(73) Patentinhaber: HAARMANN & REIMER GMBH, 37601 Holzminden (DE)
(72) Erfinder: Surburg, Horst Dr., 37603 Holzminden (DE); Sommer, Horst Dr., 37603 Holzminden (DE); Lambrecht, Stefan Dr., 37603 Holzminden (DE); Wörner, Peter, 37603 Holzminden (DE); Güntert, Matthias Dr., 37603 Holzminden (DE); Kindel, Günter, 37671 Höxter (DE); Koppe, Volkmar, 37603 Holzminden (DE)
(74) Vertreter: Eisenführ, Speiser & Partner

(56) Entgegenhaltungen:
- EP-A- 0 761 629
- PATENT ABSTRACTS OF JAPAN vol. 013, no. 021 (C-560), 18. Januar 1989 & JP 63 227546 A (KURARAY CO LTD), 21. September 1988
- DUNLOP, R. W. ET AL: AUST. J. CHEM., Bd. 32, Nr. 12, 1979, Seiten 2735-2739, XP002087973

## Beschreibung

Die Erfindung betrifft 8-Ocimenylester, Verfahren zu ihrer Herstellung und ihre Verwendung als Riech- und Aromastoffe.

In der Parfüm- und Aromenindustrie besteht nach wie vor ein verstärkter Bedarf an Stoffen, die in der Lage sind, in Parfüm- und Aromakompositionen solche Naturstoffe ganz oder teilweise zu ersetzen, die wegen ihrer mühsamen Art der Gewinnung teuer und nicht uneingeschränkt verfügbar und darüber hinaus in ihren Eigenschaften beträchtlichen naturbedingten Qualitätsschwankungen unterworfen sind.

Besonders interessant sind solche Stoffe dann, wenn sie nicht nur herausragende organoleptische (d.h. nur mit den Sinnen wahrnehmbare) Eigenschaften besitzen, sondern wenn sich mit ihnen auch wegen ihrer Stärke und Ausgiebigkeit bemerkenswerte Effekte bei geringsten Dosierungen erzielen lassen.

Die erfindungsgemäßen Verbindungen, die Ester der 8-Ocimenole, sind mögliche Parfüm- und Aromakomponenten, auf die diese Eigenschaften besonders zutreffen.

Cis- und trans-Ocimen (Verbindungen der Formel 1 bzw. 2) sind in der Natur recht weit verbreitete Terpenkohlenwasserstoffe. Cis-Ocimen ist durch Pyrolyse von alpha-Pinen kommerziell verfügbar. Als Derivate des Ocimens haben im wesentlichen nur das 6,7-Dihydroocimen-7-ol ("Ocimenol") der Formel 3 und sein Acetat, das 6,7-Dihydroocimen-7-ylacetat ("Ocimenylacetat") der Formel 4 Bedeutung erlangt (s. S. Arctander; Perfüme and Flavor Chemicals, Selbstverlag, Montclair, N.J., 1969, Monograph 2389 und 2390.).

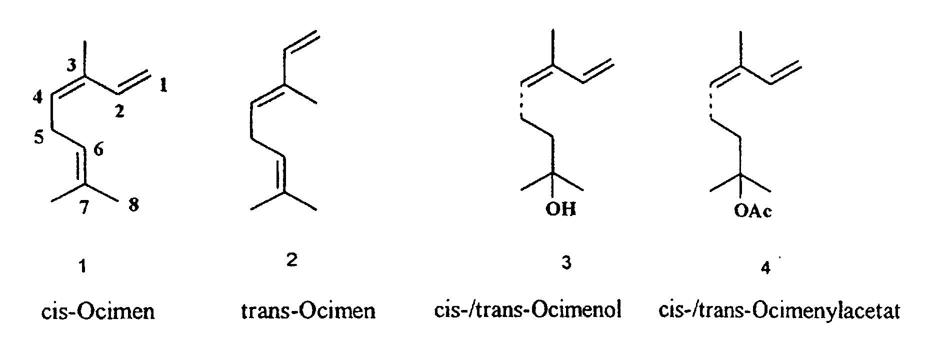

Das Ocimenol der Formel 3 besitzt einen frisch-camphrigen, Limetta-artigen Geruch mit einem süß-blumigen Unterton; das Ocimenylacetat der Formel 4 zeigt eine süßfrische, krautige, citrusartige Note.

Es wurden nun Verbindungen der Formel 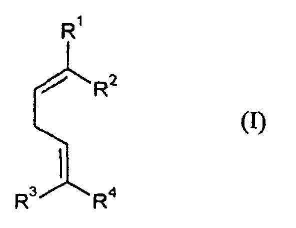 gefunden, worin
einer der beiden Reste R¹ und R² für Methyl und der andere für Vinyl steht und
einer der beiden Reste R³ und R⁴ für Methyl und der andere für 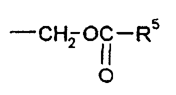 steht, wobei
- R⁵: Wasserstoff, Alkyl oder Alkenyl, insbesondere C₁-C₆-Alkyl oder C₂-C₆-Alkenyl, bedeutet.

Besonders bevorzugt sind solche Verbindungen der Formel (I), worin R⁵ für Wasserstoff, Methyl, Ethyl, n-Propyl, iso-Propyl, n-Butyl, sek.-Butyl, iso-Butyl, tert.-Butyl oder 2-Butenyl, insbesondere für Methyl, steht.

Ganz besonders bevorzugt sind Verbindungen der Formel (I), die den Formeln (Ia)-(Id) entsprechen: 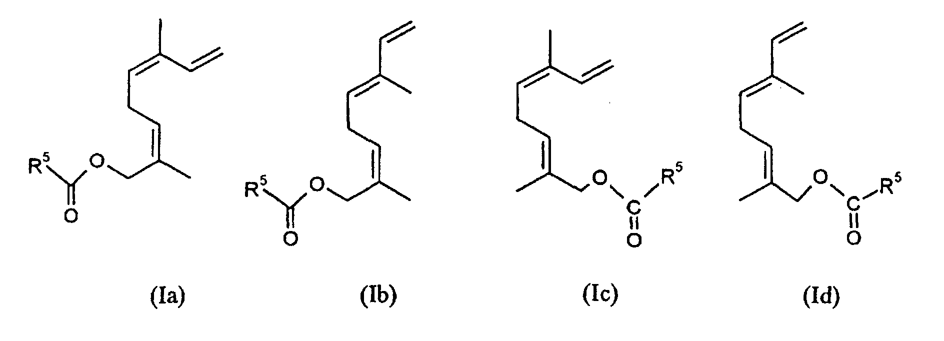 worin R⁵ die obengenannte Bedeutung hat.

Als bevorzugte dieser Formeln (Ia)-(Id) gilt die Verbindung (Ib), insbesondere mit R⁵ = Methyl.

Die erfindungsgemäßen Verbindungen ergeben gegenüber den bekannten Verbindungen 3 und 4 überraschenderweise einen völlig andersartigen Geruchseindruck.

Die Erfindung betrifft weiterhin ein Verfahren zur Herstellung der Verbindungen der Formel (I), dadurch gekennzeichnet, daß man Verbindungen der Formel 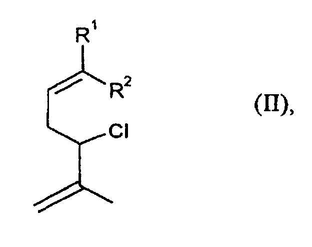 worin
R¹ und R² die oben angegebenen Bedeutungen haben,
mit dem Salz, insbesondere einem Alkalisalz wie Na- oder K-Salz, einer aliphatischen Carbonsäuren, insbesondere einer C₂-C₇-Carbonsäure, umsetzt.

Bevorzugt wird das erfindungsgemäße Verfahren in einem organischen Lösungsmittel durchgeführt, insbesondere in einem dipolar-aprotischen Lösungsmittel. Als solche kommen beispielsweise in Frage Dimethylformamid, Dimethylsulfoxid, N-Methylpyrrolidon, Diethylglycoldimethylether sowie deren Mischungen. Die Umsetzung erfolgt dabei vorzugsweise bei einer Temperatur von 50 bis 150°C, insbesondere bei 90 bis 120°C, gegebenenfalls unter Druck.

Das erfindungsgemäße Verfahren, ausgehend von den Verbindungen der Formel (II) wird vorzugsweise in Gegenwart eines Alkalimetalliodides oder elementarem Iod, insbesondere in Gegenwart von NaI durchgeführt, wobei dieser Zusatz vorzugsweise in katalytischen Mengen, insbesondere in einer Menge von 0,01 bis 10 Gew.-% bezogen auf II, eingesetzt wird.

Bei dem erfindungsgemäßen Verfahren kann die Verbindung der Formel (II) als E-oder Z-Isomer oder als beliebiges E/Z-Gemisch eingesetzt werden.

Verwendet man das E/Z-Gemisch der Formel (II), also eine Mischung einer Verbindung (II), worin R¹ für Methyl und R² für Vinyl steht und einer Verbindung (II), worin R¹ für Vinyl und R² für Methyl steht, so erhält man eine Mischung, enthaltend die Verbindungen der Formeln (Ia)-(Id).

Besonders bevorzugt ist das Verfahren bei Verwendung von Natriumacetat als Salz einer aliphatischen Carbonsäure.

Die Verbindungen der Formel (II), insbesondere solche der Formeln (IIa) und (IIb) 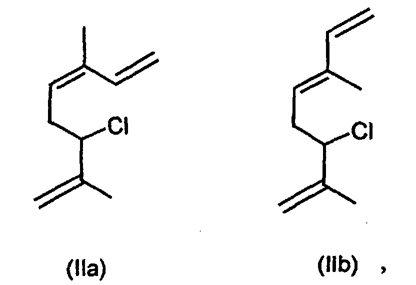 können hergestellt werden, indem man Ocimen der Formel 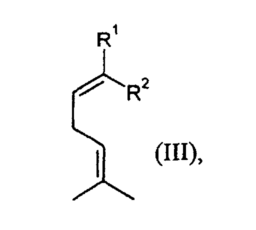 worin R¹ und R² die oben angegebenen Bedeutungen haben,
mit hypochloriger Säure oder Sulfurylchlorid (SO₂Cl₂) umsetzt.

Für die Reaktionen mit hypochloriger Säure wird diese üblicherweise in situ aus Salzen der hypochlorigen Säure durch Ansäuern erzeugt. Für die Herstellung von II hat es sich als vorteilhaft erwiesen, hypochlorige Säure aus Natriumhypochlorit-Lösung durch Ansäuern mit Essigsäure freizusetzen. Die Reaktion mit Ocimen läuft dann in einem Zweiphasen-System ab; die Anwesenheit eines Lösungsmittels ist nicht erforderlich. Die Reaktionstemperatur kann zwischen 0 und 50°C liegen; bevorzugt ist ein Temperaturbereich von 10 bis 20°C.

Die Chlorierung mit Hilfe von Sulfurylchlorid wird vorzugsweise in Gegenwart einer Base ausgeführt, vorzugsweise in Gegenwart eines Alkalimetallcarbonates wie Natrium- oder Kaliumcarbonat, das insbesondere im Überschuß, bezogen auf Sulfurylchlorid, eingesetzt wird. Als mögliche Lösungsmittel dienen gegen Sulfurylchlorid inerte Verbindungen wie Kohlenwasserstoffe, vorzugsweise n-Hexan oder n-Heptan, wie Halogenkohlenwasserstoffe, vorzugsweise Dichlormethan oder Tetrachlorkohlenstoff oder wie Ether, vorzugsweise Methyl-tert.-butylether. Die Reaktionstemperatur liegt insbesondere bei 0 bis 50°C, bevorzugt ist ein Bereich von 20 bis 30°C.

Das Ocimen kann dabei als cis-Ocimen der Formel (1), als trans-Ocimen der Formel (2) oder als cis/trans-Gemisch eingesetzt werden.

Bevorzugt setzt man ein Ocimen-cis/trans-Gemisch ein, wobei ein Gehalt von 50 bis 90 Gew.-% an trans-Isomeren und 50 bis 10 Gew.-% cis-Isomeren bevorzugt ist, woraus ein E/Z-Gemisch der Verbindung der Formel (II) und daraus wiederum ein Gemisch isomerer 8-Ocimenylester der Formeln (Ia)-(Id) hergestellt werden kann.

Die Erfindung betrifft weiterhin eine Verfahren zur Herstellung von Verbindungen der Formel (I), das dadurch gekennzeichnet ist, daß man Verbindungen der Formel 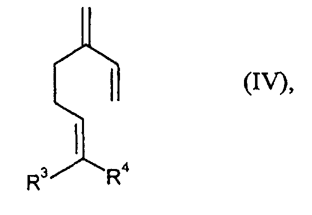 worin R³ und R⁴ die oben angegebenen Bedeutungen haben,
in Gegenwart eines Rhodium-Katalysators zu Verbindungen der Formel (I) isomerisiert.

Bevorzugt wird der Rhodiumkatalysator in einer Menge von 0,1 bis 5 Gew.-%, bezogen auf IV eingesetzt. Die Umsetzung kann mit einem organischen Lösungsmittel durchgeführt werden. Sie erfolgt aber vorzugsweise ohne Lösungsmittel. Die Isomerisierung erfolgt vorzugsweise bei einer Temperatur von 100 bis 200°C, insbesondere von 140 bis 170°C.

Verbindungen der Formel (IV) können beispielsweise dadurch hergestellt werden, daß man eine Verbindung der Formel 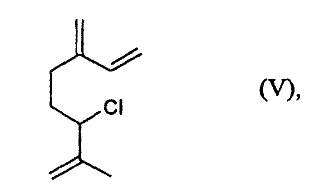 mit einem Salz, insbesondere Alkalisalz wie Na- oder K-Salz, einer aliphatischen Carbonsäure, insbesondere einer C₂-C₇-Carbonsäure, umsetzt.

Die Vorzugsformen dieser Verfahrensweise entsprechen denen, wie sie für das oben beschriebene Verfahren zur Herstellung der Verbindung I aus II angegeben sind.

Die Verbindung der Formel (V) läßt sich wiederum durch Umsetzung von Myrcen, einer Verbindung der Formel 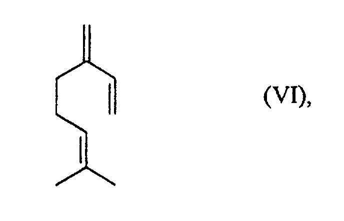 mit hypochloriger Säure herstellen, wobei auch hier die bereits für die oben beschriebene Herstellung der Verbindung II angegebenen Verfahrensbedingungen gelten.

Falls isomere Mischungen mehrerer Verbindungen der Formel (I) anfallen, insbesondere die Verbindungen (Ia) - (Id), können diese beispielsweise durch präparative Gaschromatographie getrennt oder aber als Mischungen direkt verwendet werden.

Für die reine Verbindung der Formel (Ib) mit R⁵ = Methyl zeigte sich, daß vor allem sie für den starken Galbanum-ähnlichen, Ananas-artigen Geruch der Isomerenmischung verantwortlich ist. Die anderen Isomeren der Formel (Ia), (Ic) und (Id) mit jeweils R⁵ = Methyl zeigten einen mehr oder weniger ausgeprägten fruchtigen Geruch. Die Gerüche dieser Isomeren sind besonders dazu geeignet, die Ananasartige Frische des Isomeren (Ib) abzurunden und zu unterstützen.

Erfindungsgemäß sind daher auch Mischungen enthaltend wenigstens eine Verbindung der Formel (Ib) und mindestens eine der Verbindungen (Ia), (Ic) und (Id), wobei R⁵ die oben angegebene Bedeutung hat.

Die erfindungsgemäßen Verbindungen der Formel (I) können auch ineinander überführt werden, beispielsweise durch Verseifen eines Esters der Formel (I) und Veresterung zu einer anderen Verbindung der Formel (I).

Das nach dem erfindungsgemäßen Verfahren gewonnene Gemisch der isomeren 8-Ocimenylacetate beispielsweise zeichnet sich durch einen typischen, starken grünfruchtigen, Ananas-artigen Geruch und Geschmack aus, der in seiner Tonalität sehr stark an das etherische Galbanumöl erinnert. Dieses Galbanumöl wird durch Wasserdampfdestillation aus dem Harz der Wurzeln verschiedener in Vorderasien, hauptsächlich Iran, wildwachsender Ferula-Species gewonnen. Wegen der mühsamen Art der Harz-Gewinnung gehört das Galbanumöl mit zu den kostbareren natürlichen Rohstoffen, die in der Riechstoff- und Aromenindustrie eingesetzt werden.

Auch die anderen niedermolekularen Ester der 8-Ocimenole zeigen bemerkenswerte sensorische Eigenschaften:

Die erfindungsgemäßen Verbindungen der Formel (I) bzw. ihre Isomerenmischungen eignen sich wegen ihres herausragenden organoleptischen Charakters vorzüglich als Riech- und Aromastoffe für den Einsatz in Parfümölen und Aromakompositionen. Besonders überraschend ist, daß sie der betreffenden Komposition eine bemerkenswerte natürliche Frische und Strahlung verleihen, ohne daß der eigentliche Charakter die Komposition dabei zu stark dominiert , d.h. die grün-fruchtige Note zu stark in den Vordergrund tritt. Insgesamt wird also der sensorische Gesamteindruck von Riechstoff- und Aromakompositionen durch Zugabe der erfindungsgemäßen Verbindungen in beachtlichem Maße verstärkt und aufgewertet.

Die erfindungsgemäßen Verbindungen der Formel (I) lassen sich sehr gut mit anderen Riech- und Aromastoffen in verschiedenen, unterschiedlichen Mengenverhältnissen zu neuartigen Parfüm- und Aroma-Kompositionen kombinieren. In Parfümkompositionen beträgt die eingesetzte Menge an Verbindungen der Formel (I) 0,01 bis 10 Gew.-%, vorzugsweise 0,05 bis 1 %, bezogen auf die gesamte Komposition.

Derartige Parfümkompositionen können nicht nur in alkoholischer Lösung als Feinparfüms verwendet werden, sondern auch zur Parfümierung von Kosmetika, z.B. Cremes, Lotionen, Aerosolen, Toilettenseifen usw., Haushaltsprodukten wie Reinigungs- und Waschmitteln, Weichspülern, Desinfektionsmitteln und Textilbehandlungsmitteln und anderer technischer Produkte dienen, wobei die Menge der Parfüm-Komposition vorzugsweise bei 0,1 bis 40 Gew.-%, insbesondere bei 0,5 bis 20 Gew.-%, bezogen auf das ganze Produkt, beträgt.

In Aroma-Kompositionen liegt die eingesetzte Menge der erfindungsgemäßen Verbindung der Formel (I) vorzugsweise bei 0,01 bis 10 Gew.-%, insbesondere bei 0,1 bis 5 Gew.-%, bezogen auf die gesamte Komposition. Derartige Aroma-Kompositionen können im gesamten Nahrungs- und Genußmittelbereich sowie in Produkten für die Mundpflege verwendet werden. Insbesondere können sie für die Aromatisierung von Fettmassen, Backwaren, Joghurt, Speiseeis, Süßwaren, Kaugummi, alkoholischen und nicht-alkoholischen Getränken, Tabak, Zahnpasta und Mundwässern verwendet werden. Die Dosierung derartiger Aroma- Kompositionen liegt vorzugsweise bei 0,0005 bis 2 Gew.-%, insbesondere bei 0,01 bis 1 Gew.-%, bezogen auf das fertige Lebens- oder Genußmittel.

Die Prozentangaben der nachfolgenden Beispiele beziehen sich jeweils auf das Gewicht.

### Beispiele

### Beispiel 1 Herstellung von 8-Ocimenylacetat aus Ocimen

a) Herstellung von (Z)- und (E)-6-Chlor-3,7-dimethyl-1,3,7-octatrien durch
   aa) Reaktion von hypochloriger Säure mit Ocimen
      Zu einem auf 10°C abgekühlten Gemisch bestehend aus 27,2 g Ocimen (E/Z-Verhältnis ca. 1:1) und 32 g Essigsäure wurden unter kräftigem Rühren während 1 h 220 g Natriumhypochlorit-Lösung mit einem Gehalt von ca. 7 % zugetropft. Die Reaktionstemperatur wurde dabei zwischen 10 und 15°C gehalten. Nach weiteren 2 h Nachrühren bei 10-15°C wurde das Reaktionsgemisch mit 200 ml Diethylether ausgerührt. Die organische Phase wurde abgetrennt, mit Wasser, Bisulfit-Lösung und Bicarbonat-Lösung gewaschen und bei Raumtemperatur unter vermindertem Druck eingeengt. Es verblieben ca. 40 g Rückstand, die ohne weitere Reinigung umsetzt wurden.
   ab) Reaktion von Sulfurylchlorid mit Ocimen
      108,8 g (0,80 mol) Ocimen (E/Z-Verhältnis ca. 2:1) und 85 g Soda werden in 500 ml Methyl-tert.-butylether vorgelegt. Bei 20 bis 27°C werden unter Kühlung in 1 bis 1,5 h 100 g (0,74 mol) Sulfurylchlorid zudosiert. Die Reaktion ist leicht exotherm. Es wird 2 bis 3 h nachgerührt, wobei eine schwache Gasentwicklung zu beobachten ist. Zur Aufarbeitung wird der Feststoff abgesaugt und das Filtrat mit 5 %iger Natriumhydroxid-Lösung und Wasser gewaschen, über Natriumsulfat getrocknet und eingeengt. Die Destillation des Rückstandes liefert bei 46 bis 72°C Kopftemperatur und 1 mbar Druck eine Fraktion von ca. 90 g mit einem Gehalt an isomeren Chlorocimenen von zusammen ca. 75 %.
b) Der unter aa) erhaltene Rückstand wurde zu einer auf 100°C erwärmten Mischung aus 82 g Natriumacetat, 3 g Natriumjodid und 300 ml Dimethylformamid gegeben. Nach 2 h Rühren bei 100°C wurde das Reaktionsgemisch abgekühlt, mit 500 ml Wasser verdünnt und mit 300 ml Hexan extrahiert. Der Hexanextrakt wurde mit Wasser gewaschen, getrocknet und das Hexan unter vermindertem Druck abgezogen. Als Rückstand verblieben ca. 30 g Rohprodukt, die zur Entfernung von dunkel gefärbten polymeren Produkten mit einem Lösungsmittelgemisch aus 95% Hexan und 5% Essigester über eine kurze Kieselgelsäule filtriert wurden. Nach Abziehen des Lösungsmittels verblieben ca. 15 g nur noch schwach gefärbtes Produkt, das durch Säulenchromatographie an 400 g Kieselgel mit 95% Hexan/5% Essigester weiter gereinigt wurde. Es wurden ca 10 g eines Isomerengemisches erhalten, das sich durch einen durchdringenden, fruchtigen an Galbanumöl erinnernden, Ananas-artigen Geruch auszeichnete.

Die Auftrennung des Isomerengemisches durch präparative Gaschromatographie und die anschließende spektroskopische Analyse ergaben, daß das Isomerengemisch zu ca. 30-35 % aus (Z3,Z6)-8-Ocimenylacetat, zu 30 - 35 % aus (E3,Z6)-8-Ocimenylacetat, zu 10-15 % aus (Z3,E6)-8-Ocimenylacetat und zu 10 - 15 % aus (E3,E6)-8-Ocimenylacetat besteht.

Charakterisierung von Verbindungen der Formeln (Ia) - (Id) mit R⁵ = Methyl
(Ia) (Z3,Z6)-8-Ocimenylacetat: leicht frisch-grün, fruchtig.
(Ib) (E3,Z6)-8-Ocimenylacetat: stark Galbanumartig, grün, fruchtig, Ananas.
(Ic)(Z3,E6)-8-Ocimenylacetat: grün, fruchtig, Ananas.
(Id) (E3,E6)-8-Ocimenylacetat: frisch, grün, estrig, blumig.

Die Zuordnung des jeweiligen Isomeren zu den einzelnen Strukturen erfolgte durch Vergleich der 13-C-NMR-Spektren (s. Tabelle 1).

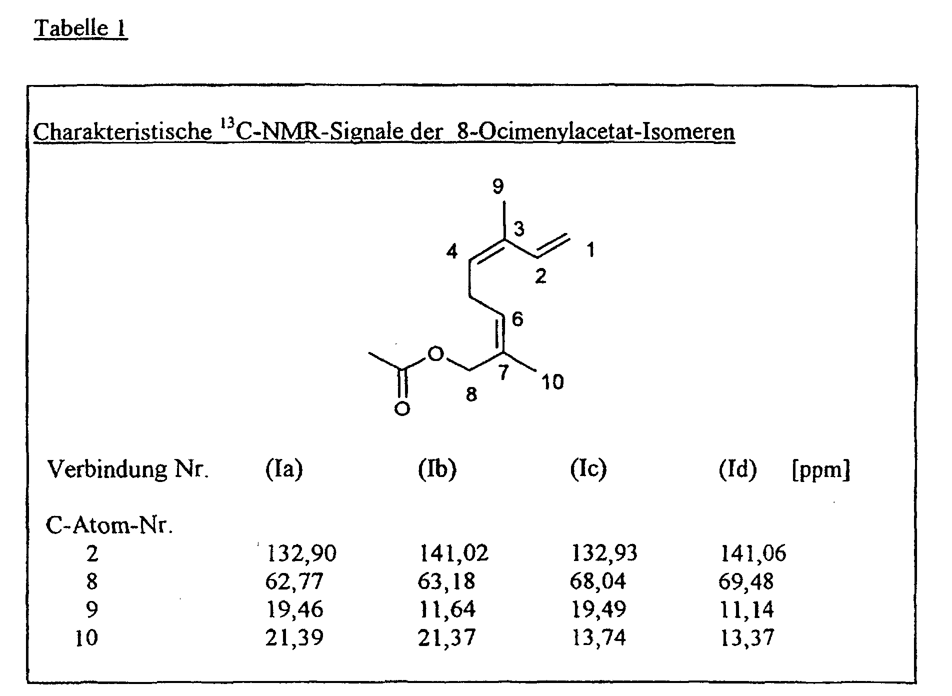

c) Analog zu dem unter Beispiel 1 b) beschriebenen Verfahren wird das nach Beispiel 1 ab) erhaltene Chlorocimen umgesetzt. Es werden nach Aufarbeitung 90 g Rohprodukt erhalten, die nach Destillation über eine kurze Kolonne bei einem Vakuum von 4 mbar und einer Kopftemperatur von 95°C 50 g eines 8-Ocimenylacetat-Gemisches ergeben, welches nach GC (gaschromatografischer Analyse) zu 25 % aus dem (Z3,Z6)-, zu 40 % aus dem (E3,Z6)-, zu 10 % aus dem (Z3,E6)- und zu 15 % aus dem (E3,Z6)-Isomeren besteht.

### Beispiel 2 Herstellung von 8-Ocimenylacetat aus Myrcen:

a) 27,2 g Myrcen wurden wie in Beispiel 1a beschrieben chloriert.
b) Das dabei erhaltene Rohprodukt wurde wie in Beispiel 1b beschrieben umgesetzt und aufgearbeitet. Es wurden etwa 15 g 8-Myrcenylacetat erhalten, nach gaschromatographischer Analyse enthaltend 60 % (Z)-Isomer und 30 % (E)-Isomer.
c) 10 g des unter b) erhaltenen Gemisches wurden mit 0,01 g BHT und 0,25 g Bis-(octadienyl)-rhodium-I-chlorid versetzt und unter Stickstoffatmosphäre für 1 h auf 160°C erhitzt. Die Aufarbeitung des Reaktionsgemisches durch Kurzwegdestillation (Kugelrohr) ergab ca. 6 g 8-Ocimenylacetat, enthaltend ca. 20 % (Z3,Z6)-8-Ocimenylacetat, 40 % (E3,Z6)-8-Ocimenylacetat, 10 % (Z3,E6)-8-Ocimenylacetat und 20 % (E3,E6)-8-Ocimenylacetat.

Aus dem 2:1-Gemisch von (Z)- und (E)-8-Myrcenylacetat entstehen so die 8-Ocimenylacetate in der angegebenen Reihenfolge in einem Verhältnis von 2 : 4 : 1: 2.

### Beispiel 3 Herstellung weiterer 8-Ocimenylester.

a) Verseifung von 8-Ocimenylacetat: 25 g eines nach Beispiel 1b gewonnenen 8-Ocimenylacetatgemisches wurden zu einer Lösung von 36,5 g Kaliumhydroxid in 265 ml Methanol gegeben. Das Reaktionsgemisch wurde 24 h bei Raumtemperatur stehengelassen. Danach wurde das Methanol unter vermindertem Druck abgezogen, der Rückstand mit Wasser aufgenommen und mit Hexan extrahiert. Nach Eindampfen des Extrakts verblieben 18,8 g Rückstand, der ohne weitere Reinigung für die im nachfolgenden beschrieben Veresterungen eingesetzt wurde.
b) 8-Ocimenylformiat:
3,2 g Acetanhydrid wurden vorgelegt und unter Kühlung mit 1,9 g Ameisensäure versetzt. Das Gemisch wird kurz auf 50° C erwärmt, dann auf 5 °C abgekühlt und 3,2 g des unter 3a erhaltenen Rohproduktes zugegeben. Nach 24 h Stehenlassen wurde das Gemisch mit Diethylether verdünnt und mit Wasser und Bicarbonatlösung gewaschen. Nach Einengen der organischen Phase verblieben 3,5 g 8-Ocimenylformiat, das in seiner Isomerenzusammensetzung dem Ausgangsmaterial entsprach. Zur Ermittlung der sensorischen Eigenschaften wurden die einzelnen Isomeren durch präparative Gaschromatographie in reiner Form isoliert.
Formiate:
(Z3,Z6)-8-Ocimenylformiat: frisch, fettig, grün, fruchtig.
(E3,Z6)-8-Ocimenylformiat: fruchtig, frisch, spritzig, Ananas, Galbanum.
(Z3,E6)-8-Ocimenylformiat: grün, fruchtig, galbanumartig.
(E3,E6)-8-Ocimenylformiat: fettig, grün, fruchtig, tropische Frucht.

| Massenspektren der 8-Ocimenylformiate | | | | | | | |
|---|---|---|---|---|---|---|---|
| (3Z,6Z) | | (3E,6Z) | | (3Z,6E) | | (3E,6E) | |
| m/z | Intens. (%) | m/z | Intens. (%) | m/z | Intens. (%) | m/z | Intens. (%) |
| 119 | 100 | 119 | 100 | 119 | 100 | 119 | 100 |
| 91 | 67 | 91 | 56 | 93 | 98 | 91 | 72 |
| 93 | 48 | 134 | 47 | 91 | 90 | 93 | 63 |
| 79 | 47 | 79 | 39 | 79 | 71 | 79 | 62 |
| 134 | 43 | 77 | 31 | 92 | 65 | 77 | 45 |
| 77 | 39 | 93 | 31 | 77 | 55 | 41 | 40 |
| 41 | 37 | 41 | 31 | 41 | 47 | 134 | 39 |
| 39 | 31 | 39 | 27 | 134 | 44 | 39 | 32 |
| 92 | 30 | 55 | 24 | 105 | 42 | 105 | 32 |
| 105 | 28 | 105 | 23 | 39 | 39 | 80 | 31 |
| 180 (M⁺) | 0,12 | 180 (M⁺) | 0,04 | 180 (M⁺) | 0,17 | 180 (M⁺) | 1 |

c) 8-Ocimenylpropionat:
3,2 g des unter 3a erhaltenen Rohproduktes und 2,5 g Pyridin wurden in 25 ml Diethylether gelöst und bei 0°C mit 2,3 g Propionsäurechlorid versetzt. Nach 24 h Stehenlassen wurde das Gemisch mit Wasser verdünnt und mit verdünnter Salzsäure, Wasser und Bicarbonatlösung gewaschen. Nach Einengen der organischen Phase verbleiben 4,2 g 8-Ocimenylpropionat, das in seiner Isomerenzusammensetzung dem Ausgangsmaterial entsprach. Zur Ermittlung der sensorischen Eigenschaften wurden die einzelnen Isomeren durch präparative Gaschromatographie in reiner Form isoliert.
Propionate:
(Z3,Z6)-8-Ocimenylpropionat: fruchtig, süßlich, fettig, grün.
(E3,Z6)-8-Ocimenylpropionat: grün, fettig, Galbanum.
(Z3,E6)-8-Ocimenylpropionat: fruchtig, süßlich
(E3,E6)-8-Ocimenylpropionat: frisch, fruchtig, süßlich, blumig.

| Massenspektren der 8-Ocimenylpropionate | | | | | | | |
|---|---|---|---|---|---|---|---|
| (3Z,6Z) | | (3E,6Z) | | (3Z,6E) | | (3E,6E) | |
| m/z | Intens. (%) | m/z | Intens. (%) | m/z | Intens. (%) | m/z | Intens. (%) |
| 119 | 100 | 119 | 100 | 57 | 100 | 119 | 100 |
| 57 | 55 | 57 | 43 | 119 | 97 | 57 | 83 |
| 91 | 39 | 134 | 35 | 91 | 48 | 91 | 39 |
| 29 | 34 | 91 | 34 | 92 | 45 | 134 | 38 |
| 134 | 33 | 29 | 29 | 29 | 44 | 29 | 38 |
| 92 | 24 | 79 | 19 | 93 | 43 | 79 | 30 |
| 93 | 23 | 105 | 18 | 134 | 38 | 93 | 29 |
| 79 | 22 | 93 | 17 | 79 | 33 | 92 | 24 |
| 105 | 22 | 92 | 16 | 105 | 31 | 105 | 22 |
| 77 | 19 | 41 | 16 | 77 | 26 | 77 | 21 |
| 208 (M⁺) | 0,12 | 208 (M⁺) | 0,08 | 208 (M⁺) | 0,22 | 208 (M⁺) | 0,12 |

d) 8-Ocimenylcrotonat:
3,2 g des unter 3a erhaltenen Rohproduktes und 2,5 g Pyridin wurden in 25 ml Diethylether gelöst und bei 0°C mit 2,6 g Crotonsäurechlorid versetzt. Nach 24 h Stehenlassen wurde das Gemisch mit Wasser verdünnt und mit verdünnter Salzsäure, Wasser und Bicarbonatlösung gewaschen. Nach Einengen der organischen Phase verblieben 2,8 g 8-Ocimenylcrotonat, das in seiner Isomerenzusammensetzung dem Ausgangsmaterial entsprach. Zur Ermittlung der sensorischen Eigenschaften wurden die einzelnen Isomeren durch präparative Gaschromatographie in reiner Form isoliert.
Crotonate:
(Z3,Z6)-8-Ocimenylcrotonat: frisch, süßlich-fruchtig.
(E3,Z6)-8-Ocimenylcrotonat: frisch, grün, Galbanum.
(Z3,E6)-8-Ocimenylcrotonat: grün, fettig, etwas frisch.
(E3,E6)-8-Ocimenylcrotonat: fruchtig, süßlich, fettig, blumig.

| Massenspektren der 8-Ocimenylcrotonate | | | | | | | |
|---|---|---|---|---|---|---|---|
| (3Z,6Z) | | (3E,6Z) | | (3Z,6E) | | (3E,6E) | |
| m/z | Intens. (%) | m/z | Intens. (%) | m/z | Intens. (%) | m/z | Intens. (%) |
| 69 | 100 | 119 | 100 | 69 | 100 | 69 | 100 |
| 119 | 95 | 69 | 81 | 119 | 55 | 119 | 75 |
| 41 | 40 | 134 | 38 | 41 | 30 | 134 | 32 |
| 91 | 37 | 41 | 38 | 91 | 26 | 41 | 31 |
| 134 | 34 | 91 | 34 | 134 | 23 | 91 | 28 |
| 39 | 24 | 39 | 23 | 92 | 21 | 79 | 19 |
| 92 | 24 | 79 | 19 | 39 | 17 | 39 | 18 |
| 105 | 22 | 105 | 19 | 79 | 17 | 93 | 17 |
| 79 | 20 | 92 | 17 | 93 | 17 | 105 | 16 |
| 93 | 19 | 77 | 17 | 105 | 16 | 92 | 16 |
| 220 (M⁺) | 0,12 | 220 (M⁺) | 0,12 | 220 (M⁺) | 0,22 | 220 (M⁺) | 0,15 |

e) 8-Ocimenylisobutyrat:
3,2 g des unter 3a erhaltenen Rohproduktes und 2,5 g Pyridin wurden in 25 ml Diethylether gelöst und bei 0°C mit 2,7 g Isobuttersäurechlorid versetzt. Nach 24 h Stehenlassen wurde das Gemisch mit Wasser verdünnt und mit verdünnter Salzsäure, Wasser und Bicarbonatlösung gewaschen. Nach Einengen der organischen Phase verblieben 4,6 g 8-Ocimenylisobutyrat, das in seiner Isomerenzusammensetzung dem Ausgangsmaterial entsprach. Zur Ermittlung der sensorischen Eigenschaften wurden die einzelnen Isomeren durch präparative Gaschromatographie in reiner Form isoliert.
Isobutyrate:
(Z3,Z6)-8-Ocimenylisobutyrat: fettig, fruchtig, grün.
(E3,Z6)-8-Ocimenylisobutyrat: grün, Galbanum, fettig, fruchtig.
(Z3,E6)-8-Ocimenylisobutyrat:frisch, fruchtig, estrig.
(E3,E6)-8-Ocimenylisobutyrat:leicht frisch, blumig.

| Massenspektren der 8-Ocimenylisobutyrate | | | | | | | |
|---|---|---|---|---|---|---|---|
| (3Z,6Z) | | (3E,6Z) | | (3Z,6E) | | (3E,6E) | |
| m/z | Intens. (%) | m/z | Intens. (%) | m/z | Intens. (%) | m/z | Intens. (%) |
| 119 | 100 | 119 | 100 | 43 | 100 | 119 | 100 |
| 43 | 61 | 43 | 50 | 119 | 98 | 43 | 77 |
| 91 | 33 | 134 | 33 | 71 | 47 | 134 | 36 |
| 134 | 31 | 91 | 29 | 92 | 42 | 71 | 33 |
| 41 | 26 | 41 | 23 | 91 | 41 | 91 | 33 |
| 92 | 24 | 105 | 17 | 134 | 37 | 41 | 28 |
| 71 | 22 | 79 | 17 | 93 | 36 | 79 | 25 |
| 105 | 21 | 92 | 17 | 41 | 35 | 93 | 24 |
| 93 | 20 | 71 | 16 | 105 | 29 | 92 | 23 |
| 79 | 20 | 93 | 16 | 79 | 29 | 105 | 21 |
| 222 (M⁺) | 0,09 | 222 (M⁺) | 0,06 | 222 (M⁺) | 0,21 | 222 (M⁺) | <0,01 |

f) 8-Ocimenylbutyrat:
3,2 g des unter 3a erhaltenen Rohproduktes und 2,5 g Pyridin wurden in 25 ml Diethylether gelöst und bei 0°C mit 2,7 g Buttersäurechlorid versetzt. Nach 24 h Stehenlassen wurde das Gemisch mit Wasser verdünnt und mit verdünnter Salzsäure, Wasser und Bicarbonatlösung gewaschen. Nach Einengen der organischen Phase verblieben 4,6 g 8-Ocimenylbutyrat, das in seiner Isomerenzusammensetzung dem Ausgangsmaterial entsprach. Zur Ermittlung der sensorischen Eigenschaften wurden die einzelnen Isomeren durch präparative Gaschromatographie in reiner Form isoliert.
Butyrate:
(Z3,Z6)-8-Ocimenylbutyrat: frisch, grün, fruchtig.
(E3,Z6)-8-Ocimenylbutyrat: grün, fruchtig, Galbanum, Ananas.
(Z3,E6)-8-Ocimenylbutyrat: leicht grün, frisch, fruchtig.
(E3,E6)-8-Ocimenylbutyrat: leicht fettig, frisch, fruchtig

| Massenspektren der 8-Ocimenylbutyrate | | | | | | | |
|---|---|---|---|---|---|---|---|
| (3Z,6Z) | | (3E,6Z) | | (3Z,6E) | | (3E,6E) | |
| m/z | Intens. (%) | m/z | Intens. (%) | m/z | Intens. (%) | m/z | Intens. (%) |
| 119 | 100 | 119 | 100 | 119 | 100 | 119 | 100 |
| 43 | 45 | 43 | 37 | 43 | 72 | 43 | 57 |
| 91 | 33 | 134 | 34 | 71 | 69 | 71 | 49 |
| 71 | 33 | 91 | 28 | 92 | 45 | 134 | 36 |
| 134 | 31 | 41 | 23 | 91 | 42 | 91 | 32 |
| 41 | 26 | 71 | 22 | 93 | 37 | 41 | 28 |
| 92 | 24 | 105 | 17 | 134 | 37 | 79 | 25 |
| 105 | 20 | 92 | 16 | 41 | 34 | 93 | 24 |
| 93 | 20 | 79 | 16 | 105 | 30 | 92 | 22 |
| 79 | 19 | 93 | 15 | 79 | 29 | 105 | 21 |
| 222 (M⁺) | 0,13 | 222 (M⁺) | 0,12 | 222 (M⁺) | 0,34 | 222 (M⁺) | 0,17 |

### Beispiel 4

Herstellung eines Parfüms mit betont blumiger Note:

Es wurden vermischt (alle Angaben in g):

| | | |
|---|---|---|
| Aldehyd C11 | 10% in Isopropylmyristat | 5 |
| Aldehyd C10 | 10% in Benzylalkohol | 5 |
| Aldehyd C11 MOA | 10% in Benzylalkohol | 3 |
| Citronellyloxyacetaldehyd | 50 % in Diethylphthalat | 2 |
| Farenal® H&R | | 3 |
| Tridecenal | 10% in Diethylphthalat | 5 |
| Hexenol, tr.-2- | 10% in Benzylalkohol | 2 |
| Vertocitral® H&R | | 1 |
| Linalylacetat | | 45 |
| Citrylal® H&R | | 5 |
| Citral | | 5 |
| Orangenöl bitter | | 5 |
| Mandarinal® (Firmenich) | | 4 |
| Lilial® (Givaudan) | | 75 |
| Lyral® (IFF) | | 75 |
| Nerolidol | | 5 |
| Profarnesol® H&R | | 5 |
| Linalool | | 45 |
| Phenylethylalkohol | | 75 |
| Geraniol | | 10 |
| Nerol | | 10 |
| Geraniumöl afrikanisch | | 5 |
| Hexylzimtaldehyd | | 50 |
| Methyldihydrojasmonat | | 15 |
| Benzylsalicylat | | 100 |
| Nonadienol, tr.-2-,cis-6- | 0,1% in Isopropylmyristat | 5 |
| Allylionon | | 3 |
| Isoraldein® 70 (Givaudan) | | 75 |
| Eugenol | | 7 |
| Cedrylacetat | | 40 |
| Vetiverylacetat | | 10 |
| Sandolen® (H&R) | | 5 |
| Galaxolid® (IFF) | 50% in Benzylbenzoat | 50 |
| Isopropylmyristat | | 245 |
| Total | | 1000 |

Durch Austausch von 2 g Isopropylmyristat gegen 2 g eines Ocimenylacetat-Isomerengemisches mit einem Gehalt von 30 - 50 % (E3,Z6)-8-Ocimenylacetat wurde die Parfümkomposition vom Typ frischer und bekam mehr Strahlung, wobei sich eine deutliche exaltierende und diffusionsteigernde Wirkung bemerkbar machte. Wert und Kostbarkeit der Parfümkomposition wurden durch die Zugabe von (E3,Z6)-8-Ocimenylacetat erheblich gesteigert.

### Beispiel 5

### Herstellung eines Fruchtaromas

Es wurden vermischt (alle Angaben in g):

| | |
|---|---|
| Aldehyd C14 sog. | 0,5 |
| Allylcapronat | 11 |
| Capronsäure | 4 |
| Citronellol | 0,3 |
| Citronenöl | 2 |
| Ethylbutyrat | 18 |
| Ethylcapronat | 2 |
| Ethylheptylat | 1 |
| Ethylpelargonat | 0,5 |
| Furaneol® (Firmenich) 15% in 1,2-Propylenglycol, | 50 |
| Isoamylbutyrat | 0,5 |
| Methyl-3-methylthiopropionat | 1,3 |
| Propionsäure | 4,5 |
| Propylenglycol, 1,2- | 904,4 |
| Total | 1000 |

Ersetzte man 10 g des Propylenglycols durch 10 g einer 1-prozentigen Lösung (in Triacetin) eines Ocimenylacetat-Isomerengemisches mit einem Gehalt von 30 - 50 % (E3,Z6)-8-Ocimenylacetat, wurde das Aroma deutlich fruchtiger und typischer in Richtung Ananas; der geschmackliche Eindruck wurde insgesamt verstärkt.

## Patentansprüche

1. Verbindungen der Formel 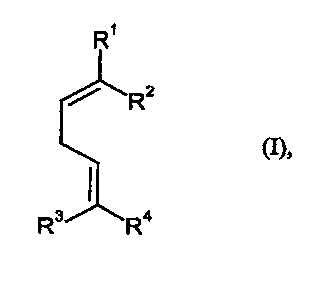 worin
einer der beiden Reste R¹ und R² für Methyl und der andere für Vinyl steht und
einer der beiden Reste R³ und R⁴ für Methyl und der andere für 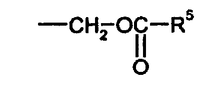 steht, wobei
R⁵ Wasserstoff, Alkyl oder Alkenyl, insbesondere C₁-C₆-Alkyl oder C₂-C₆-Alkenyl bedeutet.

2. Verbindungen gemäß Anspruch 1, worin R⁵ für Wasserstoff, Methyl, Ethyl, n-Propyl, iso-Propyl, n-Butyl, sek.-Butyl, iso-Butyl, tert.-Butyl oder 2-Butenyl, insbesondere für Methyl steht.

3. Verbindungen gemäß Anspruch 1, **dadurch gekennzeichnet, dass** sie einer der Formeln (Ia) - (Id) 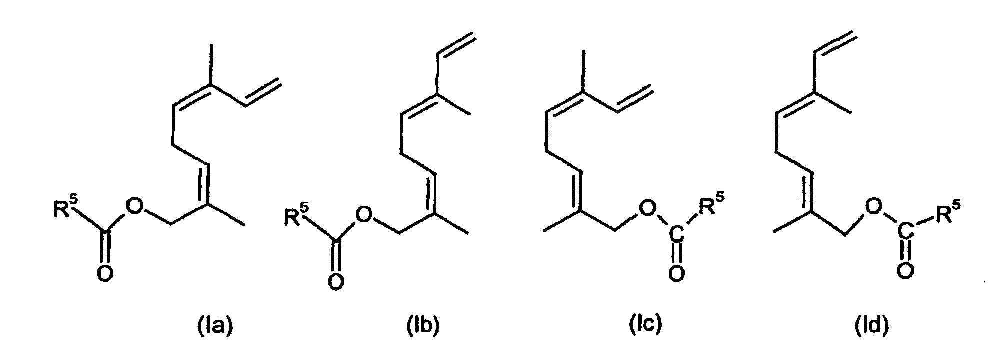 entsprechen, worin R⁵ die in Anspruch 1 angegebene Bedeutung besitzt.

4. Verfahren zur Herstellung von Verbindungen gemäß Anspruch 1, **dadurch gekennzeichnet, dass** man Verbindungen der Formel 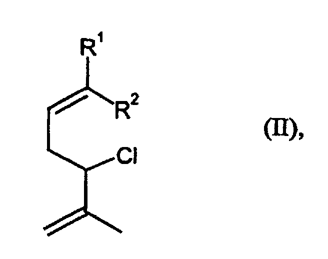 worin
R¹ und R² die in Anspruch 1 angegebenen Bedeutungen haben,
mit dem Salz, insbesondere einem Alkalisalz wie Na- oder K-Salz, einer aliphatischen Carbonsäuren, insbesondere einer C₂-C₇-Carbonsäure, umsetzt.

5. Verfahren zur Herstellung von Verbindungen gemäß Anspruch 1, **dadurch gekennzeichnet, dass** man Verbindungen der Formel 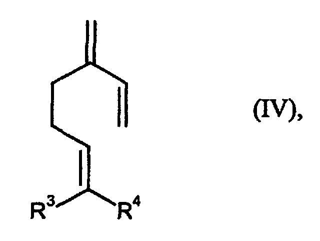 worin R³ und R⁴ die in Anspruch 1 angegebenen Bedeutungen haben, in Gegenwart eines Rhodium-Katalysators zu Verbindungen der Formel (I) isomerisiert.

6. Verwendung von 8-Ocimenylestern gemäß Anspruch 1 als Riech- und Aromastoffe.

7. Parfümöle enthaltend 8-Ocimenylester gemäß Anspruch 1.

8. Aromen enthaltend 8-Ocimenylester gemäß Anspruch 1.

## Claims

1. Compounds of the formula 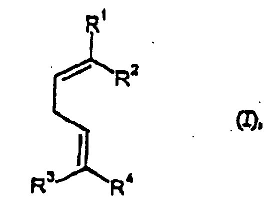 wherein
one of the two radicals R¹ and R² denotes methyl and the other denotes vinyl and
one of the two radicals R³ and R⁴ denotes methyl and the other denotes 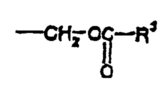 wherein
R⁵ signifies hydrogen, alkyl or alkenyl, particularly C₁-C₆ alkyl or C₂-C₆ alkenyl.

2. Compounds according to claim 1, wherein R⁵ denotes hydrogen, methyl, ethyl, n-propyl, iso-propyl, n-butyl, sec.-butyl, iso-butyl, tert.-butyl or 2-butenyl, particularly methyl.

3. Compounds according to claim 1, **characterised in that** they correspond to one of the formulae (Ia) - (Id) 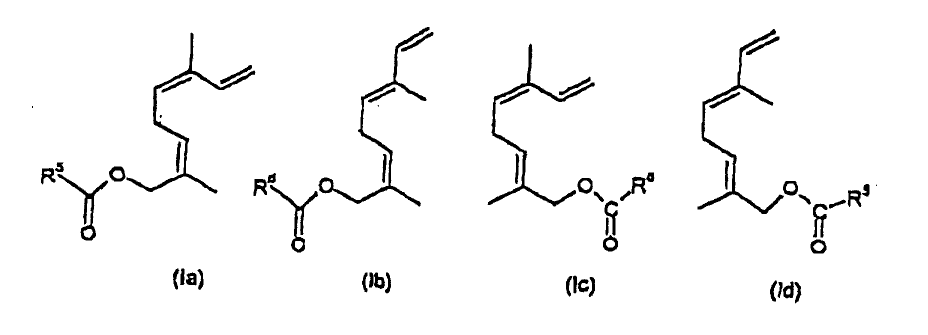 wherein R⁵ has the meaning given in claim 1.

4. Process for the production of compounds according to claim 1, **characterised in that** compounds of the formula 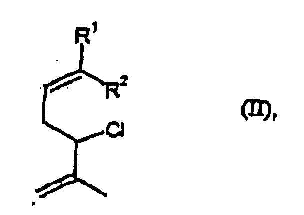 wherein
R¹ and R² have the meanings given in claim 1,
are reacted with the salt, particularly an alkali salt such as an Na or K salt, of an aliphatic carboxylic acid, particularly a C₂-C₇ carboxylic acid.

5. Process for the production of compounds according to claim 1, **characterised in that** compounds of the formula 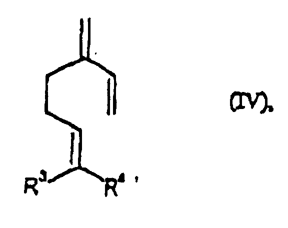 wherein R³ and R⁴ have the meanings given in claim I,
are isomerised in the presence of a rhodium catalyst to form compounds of the formula (I).

6. Use of 8-ocimenyl esters according to claim 1 as perfumes and flavourings.

7. Perfume oils containing 8-ocimenyl esters according to claim 1.

8. Flavourings containing 8-ocimenyl esters according to claim 1.

## Revendications

1. Composés de formule : 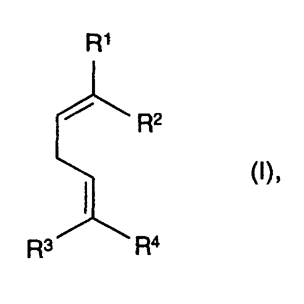 où
l'un des deux restes R¹ et R² représente méthyle et l'autre représente vinyle
et
l'un des deux restes R³ et R⁴ représente méthyle et l'autre représente 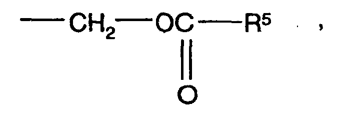 où
R⁵ représente l'hydrogène, alkyle ou alcényle, en particulier alkyle en C₁-C₆ ou alcényle en C₂-C₆.

2. Composés selon la revendication 1 où R⁵ représente l'hydrogène, méthyle, éthyle, n-propyle, isopropyle, n-butyle, sec-butyle, isobutyle, tert-butyle ou 2-butényle, en particulier méthyle.

3. Composés selon la revendication 1 **caractérisés en ce qu'**ils correspondent à l'une des formules (Ia) - (Id) 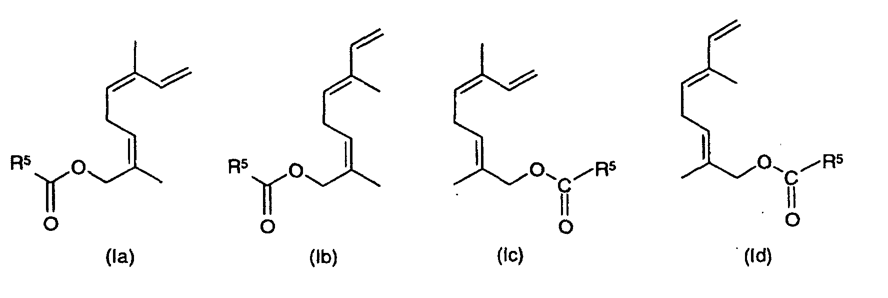 où R⁵ possède la signification indiquée dans la revendication 1.

4. Procédé de production de composés selon la revendication 1 **caractérisé en ce que** l'on fait réagir des composés de formule 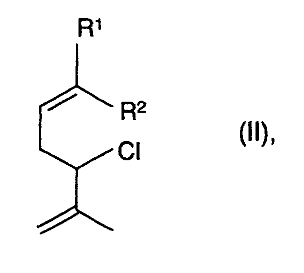 où
R¹ et R² ont les significations indiquées dans la revendication 1, avec le sel, en particulier un sel alcalin comme un sel de Na ou K, d'un acide carboxylique aliphatique, en particulier un acide carboxylique en C₂-C₇.

5. Procédé de production de composés selon la revendication 1 **caractérisé en ce que** l'on isomérise en composés de formule (I) des composés de formule 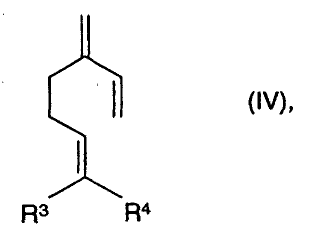 où R³ et R⁴ ont les significations indiquées dans la revendication 1, en présence d'un catalyseur au rhodium.

6. Utilisation d'esters de 8-ociményle selon la revendication 1 comme substances odorantes et aromatisantes.

7. Huiles pour parfums contenant un ester de 8-ociményle selon la revendication 1.

8. Arômes contenant un ester de 8-ociményle selon la revendication 1.
